# EUROPÄISCHE PATENTSCHRIFT

(11) **EP 2 273 467 B1**
(45) Veröffentlichungstag und Bekanntmachung des Hinweises auf die Patenterteilung: **24.12.2014**
(21) Anmeldenummer: 10010682.2
(22) Anmeldetag: 01.10.2001
(51) Int. Cl.: G08B 29/14, G01N 33/00

(54) **Gaserzeuger oder Hitzeerzeuger, Rauchgaserzeuger sowie Verfahren zum Prüfen eines Gasmelders oder eines Hitzemelders**
Gas generator or heat generator, smoke generator and method for checking a gas detector or a heat detector
Gazogène ou générateur de chaleur, générateur de fumées ainsi que procédé pour vérifier un détecteur de gaz ou de chaleur

(30) Priorität: 29.09.2000 DE 10048760; 30.01.2001 DE 10104330; 06.04.2001 DE 10117469; 09.05.2001 DE 10122572; 09.05.2001 US 289872 P; 10.05.2001 US 290133 P; 18.05.2001 US 291880 P; 15.08.2001 DE 10139033
(43) Veröffentlichungstag der Anmeldung: 12.01.2011
(62) Teilanmeldung aus: 01978196.2
(73) Patentinhaber: Tormaxx GmbH, 41199 Mönchengladbach (DE)
(72) Erfinder: Koch, Hubert, 41199 Mönchengladbach (DE)
(74) Vertreter: Castell, Klaus

(56) Entgegenhaltungen:
- EP-A2- 0 890 837
- DE-A1- 2 238 733
- DE-B- 1 187 165
- US-A- 3 729 979

## Beschreibung

Die Erfindung betrifft einen Gaserzeuger sowie ein Verfahren zum Prüfen eines Gasmelders oder eines Hitzemelders.

Gaserzeuger sind beispielsweise aus der D1 EP 0 890 837 A und der D4 DE1187165A bekannt. Diese Gaserzeuger sind aber filigran und verstopfungsanfällig.

Der Erfindung liegt daher die Aufgabe zugrunde, einen Gaserzeuger derart weiterzuentwickeln, dass er auch für eine Rauchgasmeldeanlage langfristig sicher eingesetzt werden kann..

Die der Erfindung zugrundeliegende Aufgabe wird durch einen Gaserzeuger, mit einem ohmschen Widerstand und einem Wärmeleitkörper gelöst, wobei der Wärmeleitkörper eine Kapillarmanschette mit einem ersten Bereich, der den ohmschen Widerstand in Form eines geschlitzten Rohres umschließt, und mit einem damit entlang des Längsschlitzes verbundenen zweiten Bereich, der aus zwei ebenen, zueinander parallelen Kapillarinnenflächen gebildet ist, zwischen denen sich ein Prüfmedium mittels Kapillarkräften senkrecht zur Längsachse des ersten Bereichs zum ohmschen Widerstand hinbewegt, aufweist.

Vorteilhafte Weiterbildungen sind Gegenstand der Unteransprüche.

Unter dem Begriff "Gas" versteht man hierbei alle gasförmigen Medien, wie beispielsweise auch Gase, die Feststoffpartikel aufweisen.

Hierzu zählen also alle Rauchgase, die Feststoffpartikel aufweisen, sodass hierdurch insbesondere auch Rauchgasmelder angesprochen werden können, die zum Detektieren eines Rauchgases geschaffen sind.

Da es beispielsweise hinsichtlich Installations- oder Wartungsarbeiten schwierig sein kann, unter Druck stehende Patronen oder flüssigkeitsgefüllte Behälter zu transportieren, wird vorgeschlagen, dass der Gaserzeuger einen Festkörper aufweist, der bei Erhitzung zumindest zum Teil verdampft. Dieser Festkörper kann ein Kunststoffelement oder ein Wachs sein. Dieses Wachs wird vorzugsweise durch ein sich bei Stromdurchfluss erwärmendes Widerstandselement erhitzt, so dass bei Stromdurchfluss durch die Erwärmung ein Teil des Festkörpers verdampft. Ein derartiger wachsartiger Festkörper kann ein wasserklares Gel ohne Geruch sein. Gut geeignet sind Gele aus Kohlewasserstoffen im Bereich der Weißöle, die unter Zusatz eines Gelbildners hergestellt sind. Derartige Gele haben vorzugsweise einen Siedepunkt, der über 250 °C liegt. Der Schmelzpunkt liegt vorzugsweise bei etwa 80 °C. Dies führt dazu, dass bei derartigen Stoffen unter Raumtemperatur praktisch kein Verdampfen von Bestandteilen stattfindet. Bei der Lagerung der Stoffe oder aus dem im Prüfgerät befindlichen Vorrat kann daher eine Belastung der Umgebungsluft sicher ausgeschlossen werden.

In der Praxis werden langkettige aliphatische Kohlenwasserstoffe verwendet, von denen je Prüfvorgang etwa 1mg verdampft wird. Diese verdampfte Menge ist ohne gesundheitliche Relevanz, da aliphatische langkettige Kohlenwasserstoffe nur in hohen Konzentrationen zur mechanischen Reizung der oberen Atemwege führen. Die beschriebenen Stoffe haben darüber hinaus den Vorteil, dass sie sich in der Nähe der Stoffabgabe absetzen und die freigesetzten Mengen an Material weder zur Korrosion noch anderen negativen Einflüssen auf benachbarten elektronischen oder mechanischen Bauteilen führen.

Diese Substanz kann insbesondere bei allen in dieser Anmeldung beschriebenen Rauchgaserzeugern vorteilhaft eingesetzt werden.

Ein derartiger Gaserzeuger ist besonders einfach im Aufbau und lässt sich entweder in bekannte Gasmelder integrieren oder neben bekannten Gasmeldern anordnen. Um nur zu vorgegebenen Zeiten beispielsweise eine Rauchgaserzeugung zu bewirken, weist die Heizeinrichtung eine Fernsteuerung auf, mittels derer sie angestellt wird. Dieser einfache Aufbau eines Gaserzeugers zeigt, dass mit einfachen Mitteln der hohe Serviceaufwand insbesondere bei Rauchgasmeldeanlagen reduziert werden kann, ohne dass hierunter die Sicherheit leidet.

Um ein Überhitzen des Prüfmediums zu vermeiden, besteht die Möglichkeit, die Heizdauer der Heizeinrichtung temporär zu begrenzen, sodass für ein erneutes Aktivieren der Heizeinrichtung ein Schalter entsprechend betätigt werden muss.

Der Gaserzeuger weist einen Wärmeleitkörper auf. Beispielsweise ist der Wärmeleitkörper aus einem elektrisch leitenden Metallblech hergestellt, so dass sich die beheizbare Oberfläche erhöht. Hierbei ist die Oberfläche des Metallblechs weit aus größer als die Oberfläche eines Drahtes.

Gemäß einer weiteren Ausführungsform, ist es vorteilhaft, wenn der Wärmeleitkörper ein poröses Material aufweist. Die Porosität des Materials erlaubt es, eine weitere Substanz nach Art eines Schwammes aufzusaugen, so dass die Substanz mit dem Wärmeleitkörper im Bereich der Poren in einem innigen Kontakt steht. Wird der Wärmeleitkörper bzw. der elektrische Widerstand erhitzt, verdampft die in dem porösen Material eingelagerte Substanz, wodurch beispielsweise ein Rauchgas entsteht.

Hat der Wärmeleitkörper selbst keinen porösen Körper, ist es von Vorteil, wenn an dem Wärmeleitkörper ein poröses Bauteil angeordnet ist. Auch in diesem porösen Bauteil kann eine Substanz in dessen Poren eingelagert werden.

Um beispielsweise eine Rauchgaserzeugung an einer gezielten Stelle des porösen Bauteils zu erzielen und/oder um ein unkontrolliertes Austreten der Substanz aus dem porösen Bauteil zu verhindern, ist es vorteilhaft, wenn der poröse Körper oder das poröse Bauteil eine Ummantelung aufweist, vorzugsweise eine hitzebeständige Folie. Durch die hitzebeständige Folie wird die durch das poröse Bauteil erhitzte Substanz daran gehindert, sich an einer unerwünschten Stelle des porösen Bauteils zu verflüchtigen.

Vorteilhafter Weise hat die Ummantelung wenigstens eine Öffnung. Beispielsweise weist die hitzebeständige Folie eine Öffnung auf, durch welche die erhitzte Substanz verdampft bzw. verraucht.

Eine besonders bevorzugte Ausführungsform sieht vor, dass der Gaserzeuger eine Schnittstelle zu einem Netzwerk aufweist. Beispielsweise ist der Gaserzeuger an ein lokales Netzwerk eines Gebäudes angeschlossen, so dass der Gaserzeuger beispielsweise von einer zentralen Einrichtung aus angesteuert werden kann. Die Schnittstelle kann hierbei sowohl kabelgebunden als auch kabellos ausgebildet sein. Es ist ebenfalls möglich, dass der Gaserzeuger nicht nur zu einem lokalen Netzwerk einen Kontakt aufweist, sondern vielmehr auch zu einem weiträumigen Netzwerk. Beispielsweise ist ein Gaserzeuger über ein weiträumiges Netzwerk mit einem zentralen Sicherheitsdienst verbunden, welcher sich nicht unmittelbar in dem Gebäude der zu prüfenden Gasmelder befindet.

Es versteht sich, dass der vorhin beschriebene Hitzeerzeuger ebenfalls mittels einer Schnittstelle mit einem Netzwerk verbunden sein kann. Hierbei ergeben sich die selben Vorteile wie bei dem Gaserzeuger.

Es wurde gefunden, dass es vorteilhaft ist, wenn das Prüfmedium lediglich eine Masse von weniger als 5 g, vorzugsweise von weniger als 1 g, aufweist. Vorteilhafterweise benötigt der erfindungsgemäße Rauchgaserzeuger für eine Prüfsequenz lediglich ca. 0,001 g des gelartigen Prüfmediums, sodass mit dem erfindungsgemäßen Rauchgaserzeuger bis zu 600 Prüfungen durchgeführt werden, ohne den Rauchgaserzeuger mit einem Prüfmedium neu zu befüllen. Dies entspricht bei einem monatlichen Prüfzyklus einer Einsatzdauer von ca. 40 Jahren. Vorteilhafterweise lässt sich der Rauchgaserzeuger mit einem neuen Prüfmedium nachfüllen. Durch die geringe Menge an Prüfmedium verringert sich das Gewicht des Rauchgaserzeugers erheblich.

Eine vorteilhafte Ausgestaltung der Erfindung sieht vor, dass ein Gaserzeuger, insbesondere ein Gaserzeuger zum Simulieren eines realen Rauchgases mit einer Sammeleinrichtung für das erzeugte Rauchgas ausgestattet ist, wobei die Sammeleinrichtung wenigstens ein Mittel zum Verschließen aufweist. Eine Sammeleinrichtung für das erzeugte Rauchgas ist deshalb vorteilhaft, da der Gaserzeuger unter anderem bedingt durch seine eingeschränkte Baugröße selten in der Lage ist, derart viel Rauchgas in einem kurzen Zeitraum zu produzieren, dass die Menge an produzierten Rauchgases meist nicht ausreicht, um einen Gasmelder zu aktivieren. Umfasst der Gaserzeuger nun eine Sammeleinrichtung, wird beispielsweise in dieser über einen längeren Zeitraum das erzeugte Rauchgas gesammelt und erst nach einem bestimmten Zeitraum in einem Vorgang freigelassen.

Um das Rauchgas zumindest solange in der Sammeleinrichtung zurückzuhalten, bis ein genügend großes Volumen an Rauchgas hergestellt ist, benötigt die Sammeleinrichtung wenigstens ein Mittel zum Verschließen derselben. Dies ist beispielsweise eine einfache Klappe oder ein Ventil oder ähnliches. Es ist hierbei nicht zwingend erforderlich, dass das Mittel zum Verschließen die Sammeleinrichtung zu 100% dicht abschließt.

Vorteilhaft ist es, wenn die Mittel zum Verschließen einen Draht aufweisen, dessen Gestalt temperaturabhängig ist. Mittels eines solchen Drahtes ist es möglich, beispielsweise die Klappe derart zu betätigen, dass sie die Sammeleinrichtung verschließt bzw. zumindest teilweise öffnet. Hierbei ist der Draht vorzugsweise der Gestalt, dass er durch einen elektrischen Strom und die damit einhergehende Temperaturerhöhung verkürzt bzw. verlängert werden kann.

Besonders vorteilhaft ist es, wenn die Mittel zum Verschließen einen Nitiuol-Draht aufweisen. Der Nitiuol-Draht zieht sich beispielsweise bei einem Durchströmen mit 2,5 Volt in Folge der daraus resultierenden Erwärmung zusammen und dehnt sich beim Abkühlen wieder entsprechend aus. Hierbei kann das Zusammenziehen des Drahtes zum Öffnen der Klappe verwendet werden.

Es versteht sich, dass alternativ hierzu verständlicherweise jeder anderer Aktor oder beispielsweise auch ein Linearmotor verwendet werden kann.

Nach der Erfindung ist weiter vorgeschlagen, dass die Sammeleinrichtung eine Rauchzuflussöffnung aufweist. Mittels dieser Rauchzuflussöffnung ist es möglich, dass ein erzeugter Rauch beispielsweise von der Wärmekammer des Gaserzeugers in die Sammeleinrichtung gelangt.

Eine bevorzugte Ausführungsvariante sieht vor, dass die Sammeleinrichtung wenigstens eine Eintrittsöffnung und/oder wenigstens eine Austrittsöffnung aufweist.

Unter den Begriffen "Eintrittsöffnung" bzw. "Austrittsöffnung" versteht man hierbei jegliche Öffnung, durch welche beispielsweise ein Volumenstrom in die Sammeleinrichtung hinein- bzw. hinausströmen kann. Durch die Eintrittsöffnung bzw. durch die Austrittsöffnung ist gewährleistet, dass das Rauchgas an sich durch einen Unterdruck oder mittels eines weiteren Gases aus der Sammeleinrichtung herausgelangt. Es ist auch möglich, dass in der Sammeleinrichtung kurzzeitig ein Überdruck hergestellt wird, der das Rauchgas aus der Sammeleinrichtung "herausschießt".

Bevorzugt strömt durch die Eintritts- bzw. Austrittsöffnung ein Luftvolumen.

Besonders vorteilhaft ist es, wenn die Sammeleinrichtung einen Gasmelder aufweist. Um beispielsweise den Gaserzeuger hinsichtlich einer Rauchgasentwicklung zu überprüfen, ist es vorteilhaft, wenn der entsprechende Gasmelder unmittelbar in der Sammeleinrichtung des Gaserzeugers angeordnet ist. Beispielsweise sind die Eintrittsöffnung und die Austrittsöffnung im Normalbetrieb geöffnet, so dass ein Luftvolumen, welches beispielsweise in einem Klimaanlagensystem zirkuliert, die Sammeleinrichtung zumindest teilweise durchströmt. Hierdurch ist unter anderem gewährleistet, dass der Gasmelder im Normalbetrieb ebenfalls durch das Luftvolumen bzw. durch einen Teil des Luftvolumens durchströmt wird.

Unter dem Begriff "Normalbetrieb" versteht man hierbeibeispielsweise einen stationären Betrieb einer Anlage ohne Störfall.

Um den Gasmelder nun zu überprüfen, werden die Eintrittsöffnung und die Austrittsöffnung entsprechend der vorhergehend beschriebenen Techniken (Klappe,Ventil) verschlossen, so dass ein durch den Gaserzeuger erzeugtes Rauchgas nicht aus der Sammeleinrichtung entweichen kann und somit unmittelbar in Kontakt mit dem Gasmelder kommt. Nach einer erfolgreichen Überprüfung des Gasmelders werden die Eintrittsöffnung und die Austrittsöffnung der Sammeleinrichtung wieder freigegeben, so dass die Sammeleinrichtung durch den Luftvolumenstrom freigeblasen wird.

Eine vorteilhafte Weiterbildung der Erfindung sieht vor, dass die Sammeleinrichtung ein Rohr aufweist. Beispielsweise ist das Rohr der Gestalt, dass es an einem Lüftungskanal angebracht werden kann, wobei dann ein Teil des durch den Lüftungskanal strömenden Volumenstroms das an dem Lüftungskanal angeordnete Rohr durchströmt. Hierbei bildet das Rohr einen "Bypass" zu dem eigentlichen Lüftungskanal. Dies eignet sich besonders gut für ein nachträgliches Ausrüsten eines Lüftungskanals mit einer Feuermeldeanlage.

Besonders vorteilhaft ist es, wenn die Sammeleinrichtung einen veränderlichen Querschnitt aufweist. Mittels des veränderlichen Querschnittes werden beispielsweise unterschiedliche Drücke und Strömungen innerhalb der Sammeleinrichtung bewirkt, was sich positiv auf die Verbreitung des Rauchgases auswirken kann.

Eine baulich besonders einfache Variante sieht vor, dass die Sammeleinrichtung einen Diffusor aufweist. Mittels des Diffusors können in der Sammeleinrichtung bzw. in dem Rohr unterschiedliche Strömungsgeschwindigkeiten und unterschiedliche Drücke hervorgerufen werden.

Eine vorzugsweise Ausführungsform sieht vor, dass die Sammeleinrichtung eine Venturidüse aufweist. Mittels der Venturidüse können in der Sammeleinrichtung ebenfalls unterschiedliche Drücke und Strömungsgeschwindigkeiten gezielt erreicht und eingesetzt werden.

Besonders vorteilhaft ist es, wenn der Gaserzeuger im Bereich einer Querschnittserweiterung angeordnet ist. Die Querschnittserweiterung bewirkt im allgemeinen eine Verringerung der Strömungsgeschwindigkeit und eine Verringerung des Druckes am Ort der Querschnittserweiterung. Ist in einem solchen Bereich bzw. in der Nähe einer solchen Querschnittserweiterung ein Gaserzeuger angeordnet, wirkt sich das besonders auf die Rauchgasausdehnung innerhalb der Sammeleinrichtung vorteilhaft aus.

Es ist nach der Erfindung weiter vorgeschlagen, dass der Gaserzeuger in einem Lüftungskanal angeordnet ist. An diesem Ort lässt sich besonders effektiv ein Rauchgas erzeugen.

Ebenfalls ist nach der Erfindung vorgeschlagen, dass der Gasmelder in einem Lüftungskanal angeordnet ist.

Die Anordnung des Gaserzeugers und/oder Gasmelders in dem Lüftungskanal baulich besonders einfach zu gestalten sowie besonders platzsparend.

Die der Erfindung zugrunde liegende Aufgabe wird weiter von einem Verfahren nach Anspruch 6 zum Prüfen eines Gasmelders oder eines Hitzemelders mit einem derartigen Gaserzeuger gelöst.

Ein Ausführungsbeispiel einer Rauchgasmeldeanlage und Ausführungsbeispiele verschiedener Rauchgaserzeuger sind in der Zeichnung dargestellt und werden im Folgenden näher erläutert. Es zeigt
- Figur 1: eine Rauchgasmeldeanlage aus Rauchgasdetektor und Rauchgaserzeuger,
- Figur 2: einen nicht erfindungsgemäßen Rauchgaserzeuger mit einem erwärmbaren benetzbaren Stab,
- Figur 3: einen nicht erfindungsgemäßen Rauchgaserzeuger mit einem beheizbaren Kapillarrohr,
- Figur 4: einen nicht erfindungsgemäßen Rauchgaserzeuger mit Rauchgaspatronen,
- Figur 5: einen nicht erfindungsgemäßen Rauchgaserzeuger mit einem Flüssigkeitssprühbehälter,
- Figur 6 bis 9: unterschiedliche nicht erfindungsgemäße Ausführungsbeispiele von Hitzemeldern und entsprechenden Hitzeerzeuger,
- Figur 10: eine zweigeteilte Wärmekammer,
- Figur 11: eine teilweise geschnittene Wärmekammer,
- Figur 12: schematisch eine Akku-Stromquelle,
- Figur 13: erfindungsgemäße Gaserzeuger mit einem Widerstand und einer Manschette mit Kapillarflächen,
- Figur 14: eine schematische Darstellung eines Rauchgaserzeugers,
- Figur 15: einen alternativen Rauchgaserzeuger in Wechselwirkung mit einem Rauchgasmelder,
- Figur 16 und 17: einen Rauchgasmelder in einem Lüftungskanal,
- Figur 18 und 19: einen alternativen Rauchgasmelder in einem Lüftungskanal,
- Figur 20: einen weiteren Rauchgasmelder in einem Bypass eines Lüftungskanals,
- Figur 21: einen Rauchgasmelder in einem alternativen Bypass eines Lüftungskanals und
- Figur 22: ein Verfahren zum Prüfen wenigstens eines Gasmelders.

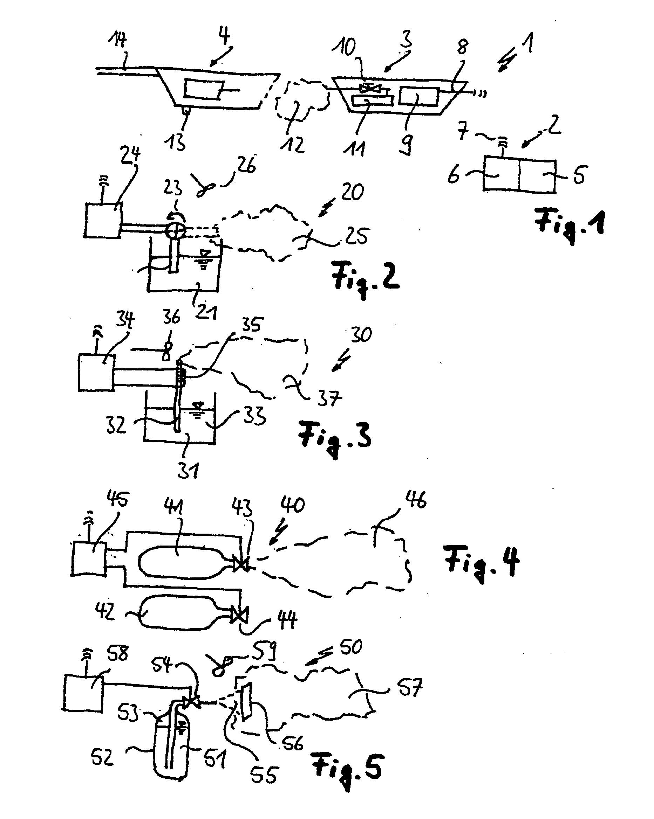

Die in Figur 1 dargestellte Rauchgasmeldeanlage 1 besteht aus dem Sender 2, dem Rauchgaserzeuger 3 und dem Rauchgasdetektor 4, In dieser Anordnung sind der Rauchgaserzeuger 3 und der Rauchgasdetektor 4 derart zueinander fixiert, dass sie miteinander in Wirkverbindung stehen.

Der Sender 2 besteht aus einer Zeituhr 5 und einer Sendeeinrichtung 6. Die Zeituhr 5 erlaubt es somit, zu bestimmten vorgegebenen Zeiten wie beispielsweise jeden Monat die Sendeanlage 6 auszulösen, so dass diese ein Signal 7 abgibt. Dieses Signal 7 wird von der Antenne 8 des Rauchgaserzeugers aufgenommen und die Empfangsanlage 9 bewirkt ein Öffnen des Ventils 10, so dass Rauchgas aus dem Reservoir freigegeben wird. Die dabei entstehende Rauchgaswolke 12 dehnt sich aus und wird von dem in der Nähe angeordneten Detektor 4 erfasst. Der Detektor 4 lässt im Detektionsfall eine Leuchtdiode 13 blinken und gibt über die Leitung 14 ein Steuersignal ab. Mit diesem Steuersignal kann beispielsweise ein Rolltor geschlossen werden oder eine Sprinkleranlage angeschaltet werden.

Die Sendeanlage 2 ist leicht zugänglich angeordnet, so dass sie auch manuell bedienbar ist. Der Rauchgaserzeuger 3 und der Rauchgasdetektor 4 sind vorzugsweise im Deckenbereich von Gebäuden nahe beieinanderliegend angeordnet, so dass sichergestellt ist, dass die Rauchgaswolke 12 vom Rauchgasdetektor 4 wahrgenommen wird.

Je nach Anwendungsfall kann anstelle eines Rauchgases auch jedes andere Gas erzeugt werden, um einen entsprechenden Gasdetektor 4 auf dessen Wirksamkeit zu prüfen.

Die Figuren 2 bis 5 zeigen verschiedene nicht erfindungsgemäßen Ausführungsbeispiele von Gaserzeugern.

Der in Figur 2 gezeigte Rauchgaserzeuger 20 besteht aus einem Flüssigkeitsreservoir 21, in das ein Stab 22 eingetaucht ist. Der Stab 22 ist entsprechend dem Pfeil 23 drehbar gelagert, so dass er um 45° in die gestrichelt dargestellte Position geschwenkt werden kann, wenn mittels des Empfängers 24 ein Funksignal wahrgenommen wird. Durch das Schwenken des Stabes 22 wird der Stab 22 derart mit einer Stromquelle verbunden, dass er sich erhitzt und die an ihm haftende Flüssigkeit verdampft. Die dabei entstehende Rauchgaswolke 25 wird mittels des Gebläses 26 in Richtung zum Rauchgasdetektor (nicht gezeigt) geblasen.

Eine alternative Ausführungsform eines Rauchgaserzeugers 30 ist in Figur 3 dargestellt. Bei diesem Rauchgaserzeuger wird aus einem Reservoir 31 mittels eines Kapillarrohres 32 Flüssigkeit 33 angesaugt. Der Empfänger 34 bewirkt, dass bei einem empfangenen Signal die Heizwendel 35 von Strom durchflossen wird und einen Teil des angesaugten Fluids 33 verdampft. Das Gebläse 36 sorgt dafür, dass die entstehende Rauchgaswolke 37 zu einem Detektor (nicht gezeigt) geblasen wird.

Die Figur 4 zeigt einen Gaserzeuger 40, der es erlaubt, zwei unterschiedliche Gasarten freizugeben. Hierzu sind zwei Gasflaschen 41, 42 vorgesehen, deren Auslässe Ventile 43, 44 aufweisen, die vom Empfänger 45 je nach empfangenem Signal geöffnet werden. Dadurch entsteht eine Gaswolke 46, die von einem Detektor (nicht gezeigt) wahrgenommen werden kann. Dieser Rauchgaserzeuger ermöglicht es somit, zu testen, ob der Detektor auch auf unterschiedliche Gasarten anspricht.

Ein weiteres Ausführungsbeispiel eines Rauchgaserzeugers 50 ist in Figur 5 gezeigt. Bei dieser Einrichtung ist eine mit einer Flüssigkeit 51 gefüllte Druckflasche 52 vorgesehen. Ein unter Druck stehendes Gaspolster 53 sorgt dafür, dass beim Öffnen des Ventils 54 ein Sprühstrahl 55 freigesetzt wird. Dieser Sprühstrahl trifft auf eine beheizte Fläche 56, so dass das aufgesprühte Fluid verdampft und eine Gaswolke 57 erzeugt. Ein Empfänger 58 ist so mit dem Ventil 54 verschaltet, dass bei einem Empfang eines Signals das Ventil 54 geöffnet wird und ein Sprühstrahl auf die Platte 56 trifft und dort verdampft. Das Gebläse 59 bläst die Gaswolke 57 zu einem Detektor (nicht gezeigt) um dort ein Signal auszulösen.

Die dargestellten Ausführungsbeispiele zeigen, dass verschiedene Gaserzeuger möglich sind, um auf einfache Art und Weise ein Gas zu erzeugen, das von einem Gasmelder wahrgenommen werden kann, um ein Signal auszulösen. Der Fachmann erkennt, dass die Möglichkeiten der Rauchgaserzeugung nicht auf die angegebenen Ausführungsbeispiele beschränkt sind.

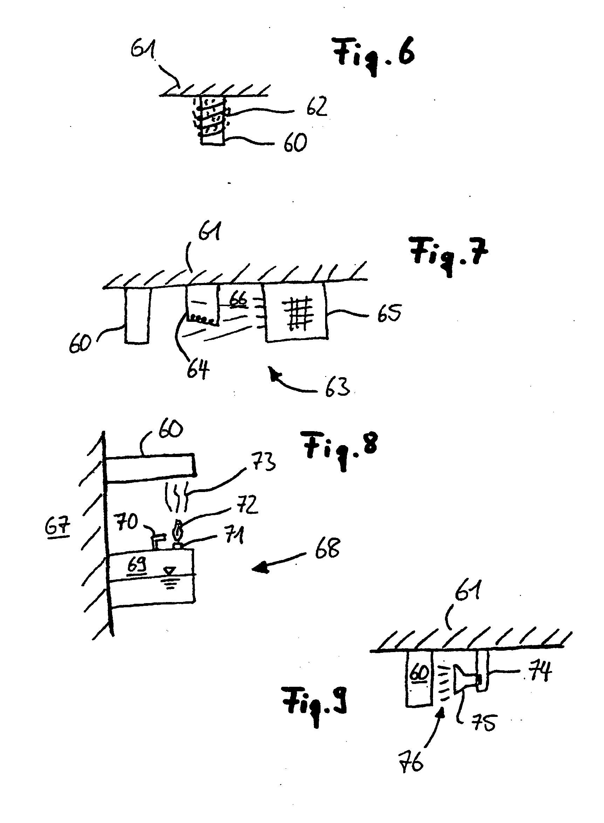

Der in Figur 6 nicht erfindungsgemäße dargestellte Hitzemelder 60 ist an einer Gebäudedecke 61 angeordnet und bildet eine körperliche Einheit mit einem Hitzeerzeuger 62, welcher in Gestalt eines Metalldrahtes 62 dargestellt ist. Soll der Hitzemelder 60 nun getestet werden, wird durch den Metalldraht 62 ein elektrischer Strom geleitet, wobei sich der Metalldraht 62 erhitzt und die erzeugte Hitze ausreicht, um den Hitzemelder 60 zu aktivieren.

Der in Figur 7 nicht erfindungsgemäße gezeigte Hitzemelder 60 ist ebenfalls an der Decke 61 angeordnet. In der Nähe des Hitzemelders 60 ist eine Hitzeerzeugungsanlage 63 angeordnet, wobei die Hitzeerzeugungsanlage 63 aus einer Heizwendel 64 und einem Gebläse 65 besteht. Bei einem Test des Hitzemelders 60 wird die Glühwendel 64 aktiviert, sodass die Glühwendel 64 Hitze erzeugt. Diese Hitze wird dann mittels des Gebläses 65 und einer Luftströmung 66 an den Hitzemelder herangetragen, sodass diese die Hitze detektiert.

Ebenfalls ist es möglich, den Hitzemelder 60 an einer vertikal verlaufenden Fläche 67 anzuordnen. Unter dem Hitzemelder 60 befindet sich ein Hitzeerzeuger 68, wobei der Hitzeerzeuger 68 einen Fluidbehälter 69 sowie eine Zündeinrichtung 70 aufweist. Durch eine Düse 71 strömt hierbei ein brennbares Medium, welches durch die Zündeinrichtung 70 entzündet wird, sodass ein Flamme 72 eine Hitze 73 entwickelt, wobei die aufsteigende Hitze 73 dem Hitzemelder 60 auslöst.

In einem weiteren Ausführungsbeispiel ist der Hitzemelder 60 wiederum an der Decke 61 angeordnet. In unmittelbarer Nähe des Hitzemelders 60 ist ein Hitzeerzeuger 74 angeordnet. Der Hitzeerzeuger 74 weist einen Wärmestrahler 75 in Form einer Infrarotlampe auf. Ist die Infrarotlampe aktiviert, erzeugt sie eine Wärmestrahlung 76, die den Hitzemelder 60 aktiviert.

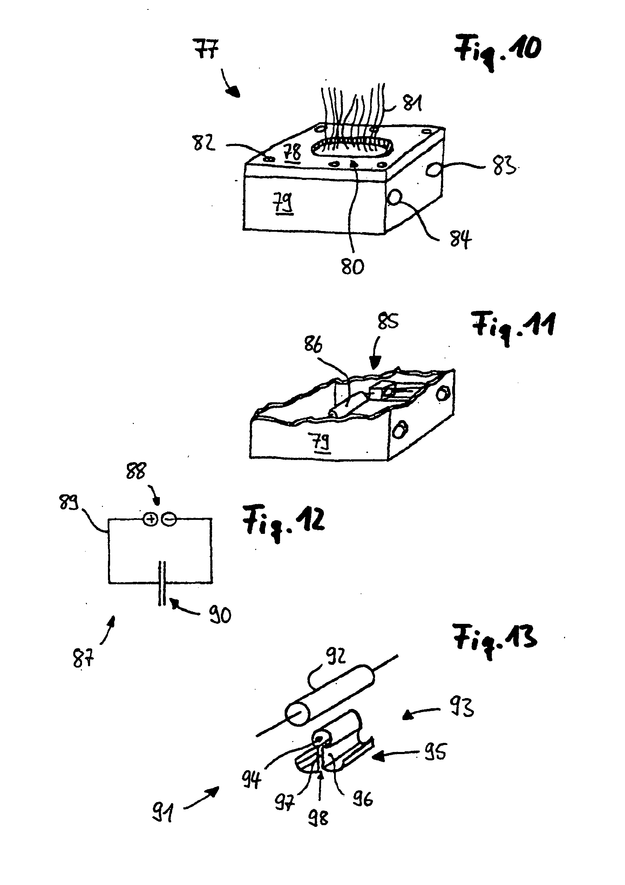

Die in Figur 10 dargestellte zweigeteilte Wärmekammer 77 weist einen Verschlussdeckel 78 und ein Basisgehäuse 79 auf. Der Verschlussdeckel 78 hat in seiner Mitte eine Öffnung 80, durch welche ein erzeugtes Rauchgas 81 aufsteigt. Des Weiteren ist der Verschlussdeckel 78 mittels einer Vielzahl von Schrauben 82 mit dem Basisgehäuse 79 verschraubt. Das Basisgehäuse 79 hat an einer seiner Seiten zwei Bohrungen 83 und 84, welche Steckkontakte für einen elektrischen Anschluss (hier nicht dargestellt) aufnehmen.

Im Inneren des Basisgehäuses 79 ist eine Heizeinrichtung 85 angeordnet, wobei die Heizeinrichtung 85 einen Widerstand 86 aufweist.

In der Figur 12 ist prinzipiell der einfache Aufbau eines elektrischen Stromkreises 87 des erfindungsgemäßen Rauchgaserzeugers dargestellt. Als Stromquelle dient hierbei ein Akkumulator 88, welcher mittels einer Drahtverbindung 89 mit einem Wärmeleitkörper 90 (Kondensator, ohmischer Widerstand) verbunden ist.

Die Anordnung 91 der Figur 13 zeigt den erfindungsgemäßen Gaserzeuger mit einem ohmschen Widerstand 92 sowie einer Kapillarmanschette 93 nach Anspruch 1. Der Außendurchmesser des ohmschen Widerstandes 92 entspricht in etwa dem Innendurchmesser der Kapillarmanschette 93 in einem Bereich 94 mit seinem unteren Bereich 95 ist die Kapillarmanschette 93 in einem gelartigen Prüfmedium (hier nicht dargestellt) angeordnet, wobei sich das gelartige Prüfmedium im Innenbereich zwischen den Kapillarflächen 96 und 97 mittels Kapillarkräfte in Pfeilrichtung 98 zwischen den beiden Kapillarinnenflächen 96 und 97 zu dem ohmschen Widerstand 92 hinbewegt.

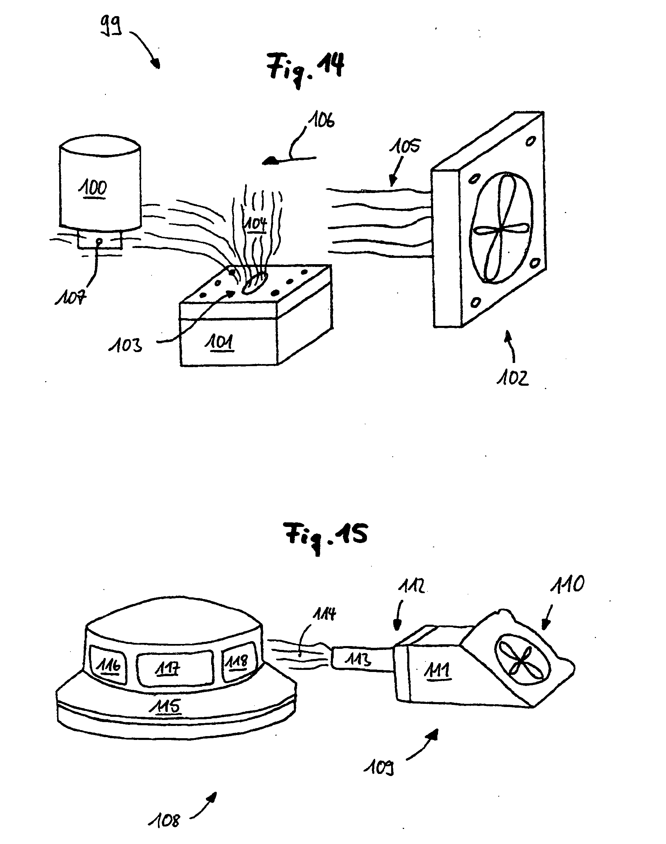

Eine Anordnung 99 der Figur 14 umfasst einen Rauchgasmelder 100, eine Wärmekammer 101 und einen Ventilator 102. Durch eine Öffnung 103 der Wärmkammer 101 gelangt ein Rauchgas 104 in die unmittelbare Umgebung der Anordnung 99. Der Ventilator 102 bläst einen Luftstrom 105 in Pfeilrichtung 106. Hierbei wird das Rauchgas 104 mit der Luftströmung 105 mitgeführt und von einem Detektor 107 des Rauchgasmelders 100 registriert, wodurch ein Alarmsignal injiziert wird.

Ist die Prüfung des Rauchgasmelders 100 abgeschlossen, wird die Entwicklung des Rauchgases 104 in der Wärmekammer 101 unterbunden, in dem die elektrische Heizeinrichtung 85 ausgeschaltet wird. Der Luftstrom 105 des Ventilators 102 bläst dem Rauchgasmelder 100, insbesondere den Detektor 107 des Rauchgasmelder 100, von den restlichen Rauchgaspartikeln des Rauchgases 104 frei.

In dem Ausführungsbeispiel 108 ist eine Rauchgaserzeuger 109 und ein Ventilator 110 in einem Gehäuse 111 zusammen angeordnet. Das Gehäuse 111 weist in seinem vorderen Bereich 112 ein Rohr 113 auf, durch welches ein durch den Rauchgaserzeuger 109 erzeugtes Rauchgas 114 mittels des Ventilators 110 geblasen wird. Das Gehäuse 111 ist hierbei derart relativ zu einem Rauchgasmelder 114 angeordnet, dass das Rauchgas 114 unmittelbar über Öffnungen 116, 117 und 118 bis an den Detektor des Rauchgasmelders 115 gelangt. Mittels des Rohres 113 wird ein gezieltes Anströmen des Rauchgasmelders 115 durch Rauchgas 114 des Rauchgaserzeugers 109 auf einfache Art und Weise möglich.

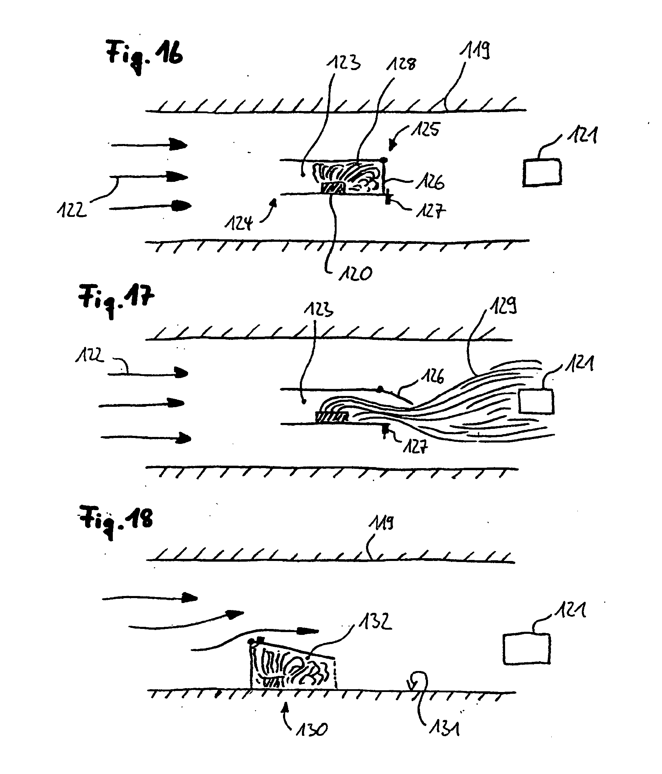

Der Lüftungskanal 119 der Figuren 16 bis 19 weist einen Rauchgaserzeuger 120 und einen Rauchgasmelder 121 auf. Der Rauchgaserzeuger 120 und der Rauchgasmelder 121 sind jeweils auf der Mittelachse des Lüftungskanals 119 hintereinander angeordnet. Durch den Lüftungskanal 119 strömt ein Luftvolumen 122. Das Luftvolumen 122 trifft zuerst auf den Rauchgaserzeuger 120 und anschließend auf den Rauchgasmelder 121.

Der Rauchgaserzeuger 120 umfasst eine Rauchgassammelkammer 123, die auf der Rauchgasmelder 121 abgewandten Seite 124 nicht verschlossen ist. An der dem Rauchgasmelder 121 zugewandten Seite 125 weist die Rauchgassammelkammer 123 einen Verschluss 126 auf. In Bereich des Verschlusses 126 befindet sich ein Verschluss 127, der vorzugsweise über einen elektrischen Impuls es ermöglicht, dass die Klappe 126 geöffnet wird.

Soll nun der Rauchgasmelder 121 geprüft werden, wird der Rauchgaserzeuger aktiviert, sodass er ein Rauchgas 128 produziert. Dieses Rauchgas 128 sammelt sich solange in der Rauchgassammelkammer 123 bis die Klappe 126 mittels des Verschlusses 127 geöffnet wird und ein konzentrierter Rauchgaszug 129 aus der Rauchgassammelkammer 123 in Richtung des Rauchgasmelders 121 entweicht. Der Rauchgaszug 129 wird mit dem Volumenstrom 122 durch den Lüftungskanal 119 mitgezogen.

Erfindungsgemäß kann sich in der Rauchgassammelkammer 123 über einen genügend langen Zeitraum derart viel Rauchgas 128 ansammeln, sodass das angesammelte Rauchgas 128 einen derart starken Rauchgaszug 129 in den Lüftungskanal 119 bewirkt, dass die Detektoren des Rauchgasmelders 121 auf diesem Rauchgaszug 129 ansprechen.

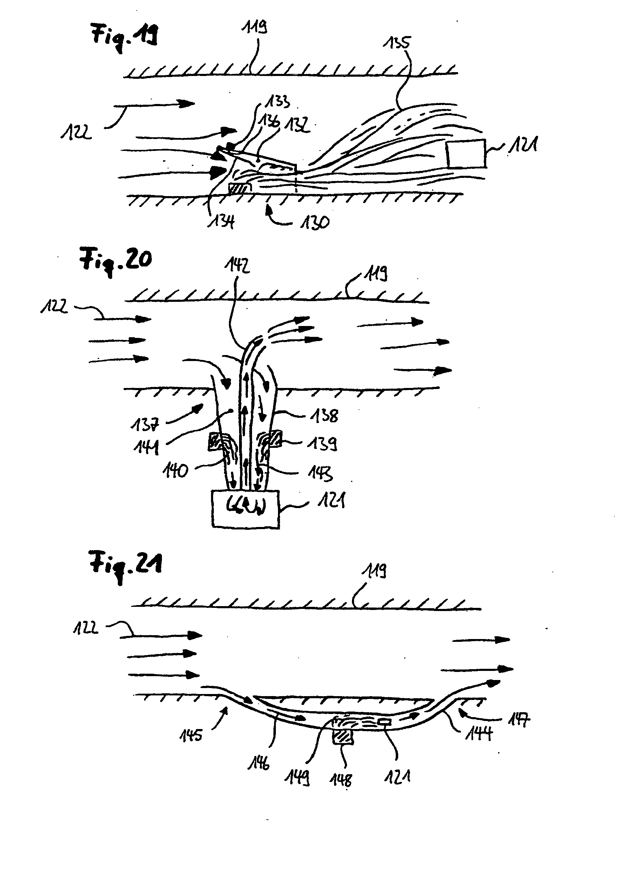

In dem Lüftungskanal 119 der Figuren 18 und 19 ist ein alternativer Rauchgaserzeuger 130 angeordnet, der bei Bedarf mit dem Rauchgasmelder 121 wechselwirkt. Der Rauchgasmelder 130 ist in diesem Ausführungsbeispiel nicht auf der Mittelachse des Lüftungskanals 119 angeordnet, sondern an der Innenseite 133 angeordnet. Der alternative Rauchgasmelder umfasst ebenfalls einen etwas größeren Hohlraum als Rauchgassammelkammer 132, in dem sich bei aktiviertem Rauchgaserzeuger 130 ein entsprechendes Rauchgas ansammelt. Hat sich in der Rauchgassammelkammer 132 genügend Rauchgas angesammelt wird mittels eines Verschlusses 133 eine Klappe 134 des Rauchgasmelders 130 geöffnet, sodass ein Volumenstrom 122 die Rauchgassammelkammer 132 zumindest teilweise durchströmen kann und hierbei das Rauchgas zu einer Rauchgaspfanne 135 mit zu dem Rauchgasmelder 121 mitreist. Der Verschluss 133 wird hierbei elektrisch angesteuert, wobei sich ein Nitiuol-Draht 136 zusammenzieht und dabei die Klappe 134 öffnet.

Der in Figur 20 dargestellte Lüftungskanal 119 weist in einem Bereich einen Durchbruch 137 auf. An dem Durchbruch 137 ist ein erstes Rohr 138 angeordnet, welches eine Verbindung zwischen dem Lüftungskanal 119 und einem Rauchgasmelder 121 bildet. An dem äußeren Rohr 138 ist ein Rauchgaserzeuger 139 angeordnet, der bei Bedarf ein Rauchgas 140 herstellen kann. Im Inneren 141 des ersten äußeren Rohres 138 befindet sich ein weiteres Rohr 142, welches ebenfalls eine Verbindung zwischen dem Lüftungskanal 119 und dem Rauchgasmelder 121 bildet.

Durch den Lüftungskanal 119 strömt ein Luftvolumen 122. Das Luftvolumen 123 wird teilweise durch das Innere 141 des Rohres 138 zu dem Rauchgasmelder 121 geleitet. Von dort aus gelangt das durch das erste äußere Rohr 138 geleitete Luftvolumen mittels des zweiten inneren Rohres 142 wieder zurück in den Lüftungskanal. Da nun immer ein Teil des Luftvolumens 122 durch den Rauchgasmelder 121 mittels der beiden Rohre 138 und 142 geleitet wird, ist der Rauchgasmelder in der Lage ein Signal zu erzeugen, wenn in dem Luftvolumen 122 ein Rauchgas 140 vorhanden ist.

Soll der Rauchgasmelder 121 nun überprüft werden, wird der Rauchgaserzeuger 139 derart eingestellt, dass er ein Rauchgas 140 erzeugt. Dieses Rauchgas 140 wird nun durch ein Teilvolumen 143 des Luftvolumens 122 mittels des äußeren ersten Rohres 138 zu dem Rauchmelder 121 geführt. Dieser ist nun in der Lage das Rauchgas 140 zu detektieren und erzeugt ein entsprechendes Signal, welches ein Vorhandensein von Rauchgas 140 anzeigt.

Der Lüftungskanal 119 der Figur 21 weist einen Bypass 144 auf. Der Bypass 144 hat eine Eintrittsöffnung 145, die einen Teilvolumenstrom 146 des Volumenstroms 122 erlaubt in den Bypass 144 einzutreten und einen Auslassbereich 147, durch den der Teilvolumenstrom 146 wieder zurück in den Lüftungskanal 119 strömen kann. Der Bypass 144 weist darüber hinaus einen Rauchgaserzeuger 148 und einen Rauchgasmelder 121 auf. Des Weiteren ist der Bypass dergestalt, dass er im Bereich des Rauchgaserzeugers 148 einen größeren Querschnitt aufweist als in Bereich der Eingangsöffnung 145. Hierdurch verlangsamt sich die Strömungsgeschwindigkeit des Teilvolumenstroms 146 im Bereich des Rauchgaserzeugers 148, wodurch in diesem Bereich ein Unterdruck entsteht. Dieser bewirkt, dass ein durch den Rauchgaserzeuger 148 erzeugtes Rauchgas 149 in den Bypass 144 gesogen wird und mit dem Teilvolumenstrom 146 zu dem Rauchgasmelder 121 getragen wird.

Der Bypass 144 ist erfindungsgemäß derart an den Lüftungskanal 119 angeordnet, dass immer ein Teilvolumenstrom 146 des Volumenstroms 122 durch ihn strömt. Führt der Volumenstrom 122 ein Rauchgas mit sich, gelangt dieses mittels des Bypasses 144 zu dem Rauchgasmelder 121, der dann ein entsprechendes Signal auslöst. Soll hingegen ein Rauchgas 149 nur simuliert werden, um den Rauchgasmelder 121 auf seine Funktionstüchtigkeit hin zu überprüfen, wird das Rauchgas 149 mittels des Rauchgaserzeugers 148 erzeugt und mit dem Teilvolumenstrom 146 an den Rauchgasmelder 121 herangetragen.

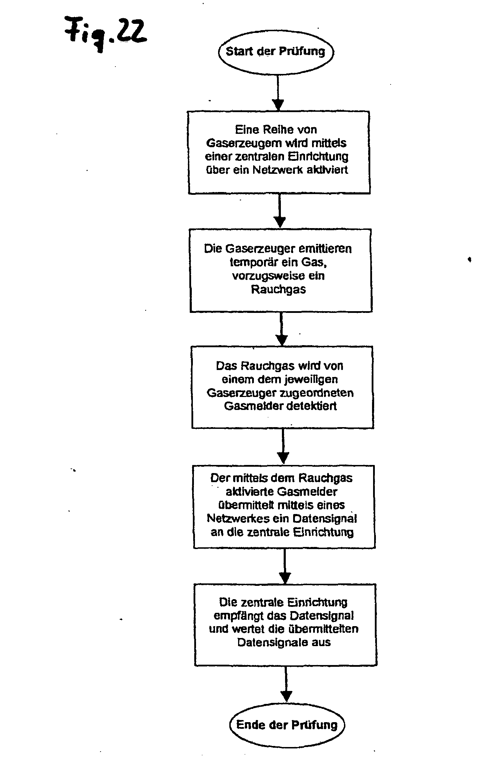

Der in der Figur 22 dargestellte Verfahrensablauf beginnt mit dem Start der Prüfung, wobei eine Reihe von Gaserzeugern mittels einer zentralen Einrichtung mittels eines Netzwerkes aktiviert wird. Daraufhin emittieren die aktivierten Gaserzeuger zumindest temporär ein Gas, wobei das Gas vorzugsweise ein Rauchgas ist. Das Rauchgas wird hierbei dem Gasmelder derart zugeführt, dass das Rauchgas von einem dem jeweiligen Gaserzeuger zugeordneten Gasmelder detektiert wird. Der mittels des Rauchgases aktivierte Gasmelder übermittelt mittels eines Netzwerkes ein Datensignal an die zentrale Einrichtung. Das Übermitteln des Datensignals zeigt an, dass der Gaserzeuger funktionsfähig ist und auf das Rauchgas anspricht. Die das Datensignal empfangene zentrale Einrichtung wertet die übermittelten Datensignale aus. Hiermit ist das Ende der Prüfung erreicht.

Erreicht ein Datensignal eines Gasmelders nicht wie vorgesehen die zentrale Einrichtung, wird der Gasmelder sowie der Gaserzeuger manuell überprüft.

Alternativ zu dem vorhergehend beschriebenen Verfahrensablauf, ist es möglich, dass ein Datensignal parallel zu der zentralen Einrichtung ebenfalls an eine weitere Einrichtung übermittelt wird. Diese Einrichtung ist beispielsweise eine Feuerwehrdienststelle. Besteht eine solche direkte Verbindung zu der Feuerwehr, ist es vorteilhaft, wenn die Feuerwehr vorzugsweise automatisch von der zentralen Einrichtung über den Zeitplan und den Ablauf der Prüfung informiert wird. Gegebenenfalls ist es möglich, dass die zentrale Einrichtung die direkte Datenleitung zu der Feuerwehr temporär deaktiviert, sodass der Feuerwehr während der Prüfung kein Datensignal übermittelt wird und somit beispielsweise die Gefahr eines Fehlalarms vermieden ist.

Nach der Prüfung wird die direkte Datenverbindung zu der Feuerwehr vorzugsweise automatisch wiederhergestellt.

Die Erfindung wurde am Beispiel eines Rauchgaserzeugers und eines Rauchgasdetektors erläutert. Sie ist jedoch auf jeden beliebigen Gasdetektor oder Hitzedetektor anwendbar, indem ein entsprechender Gaserzeuger oder Hitzeerzeuger vorgesehen wird.

## Patentansprüche

1. Gaserzeuger mit einem ohmschen Widerstand (92) und einen Wärmeleitkörper (86; 90; 93), ***dadurch gekennzeichnet, dass*** der Wärmeleitkörper (86; 90; 93) eine Kapillarmanschette (93) mit einem ersten Bereich, der den ohmschen Widerstand in Form eines geschlitzten Rohres umschließt, und mit einem damit entlang des Längsschlitzes verbundenen zweiten Bereich, der aus zwei ebenen, zueinander parallelen Kapillarinnenflächen (96, 97) gebildet ist, zwischen denen sich ein Prüfmedium mittels Kapillarkräften senkrecht zur Längsachse des ersten Bereichs zum ohmschen Widerstand hinbewegt, aufweist.

2. Gaserzeuger nach einem der vorhergehenden Ansprüche, ***dadurch gekennzeichnet, dass*** der Gaserzeuger eine Schnittstelle zu einem Netzwerk aufweist.

3. Gaserzeuger nach einem der vorhergehenden Ansprüche, ***dadurch gekennzeichnet, dass*** er ein elektrisches Gebläse aufweist.

4. Gaserzeuger nach einem der vorhergehenden Ansprüche, ***dadurch gekennzeichnet, dass*** er elektrisch auslösbar ist.

5. Gaserzeuger nach einem der vorhergehenden Ansprüche, ***dadurch gekennzeichnet, dass*** er eine Sammeleinrichtung für erzeugtes Rauchgas aufweist, die wenigstens ein Mittel zum Verschließen aufweist.

6. Verfahren zum Prüfen eines Gasmelders oder eines Hitzemelders mit einem Gaserzeuger nach einem der vorhergehenden Ansprüche, bei dem
a. wenigstens ein dezentral angeordneter Gaserzeuger mittels einer zentralen Überwachungseinrichtung aktiviert wird,
b. der Gaserzeuger hierbei ein Gas, vorzugsweise ein Rauchgas, erzeugt,
c. der Gasmelder das Rauchgas detektiert, und der Gasmelder hierbei aktiviert wird, und der Gasmelder ein Datensignal übermittelt.

## Claims

1. A gas generator with an ohmic resistance (92) and a heat conducting body (86, 90, 93), **characterised in that**, the heat conducting body (86, 90, 93) has a capillary sleeve (93) with a first region, which encloses the ohmic resistance in the form of a slit tube, and with a second region connected with the first region along the longitudinal slit, which second region is formed from two planar, parallel to one another, capillary inner surfaces (96, 97), between which a test medium moves by means of capillary forces towards the ohmic resistance at right angles to the longitudinal axis of the first region.

2. The gas generator in accordance with one of the preceding claims, **characterised in that**, the gas generator has an interface to a network.

3. The gas generator in accordance with one of the preceding claims, **characterised in that**, it has an electrical fan.

4. The gas generator in accordance with one of the preceding claims, **characterised in that**, it can be actuated electrically.

5. The gas generator in accordance with one of the preceding claims, **characterised in that**, it has a collection device for generated flue gas, which has at least one means for closure.

6. A method for testing a gas detector or heat detector with a gas generator in accordance with one of the preceding claims, in which
a. At least one decentrally arranged gas generator is activated by means of a central monitoring device,
b. The gas generator hereby generates a gas, preferably a flue gas,
c. The gas detector detects the flue gas, and the gas detector is hereby activated, and the gas detector transmits a data signal.

## Revendications

1. Gazogène avec une résistance ohmique (92) et un corps thermo-conducteur (86 ; 90 ; 93), **caractérisé en ce que** le corps thermo-conducteur (86 ; 90 ; 93) présente une manchette capillaire (93) avec une première zone, laquelle entoure la résistance ohmique en forme d'un tube fendu, et avec une deuxième zone en liaison avec celle-ci le long de la fente longitudinale, laquelle est formée par deux surfaces internes capillaires (96, 97) planes, parallèles entre elles, entre lesquelles un milieu d'essai se déplace à l'aide de forces de capillarité, perpendiculairement à l'axe longitudinal de la première zone, vers la résistance ohmique.

2. Gazogène selon l'une des revendications précédentes, **caractérisé en ce que** le gazogène présente une interface avec un réseau.

3. Gazogène selon l'une des revendications précédentes, **caractérisé en ce qu'**il présente une soufflante électrique.

4. Gazogène selon l'une des revendications précédentes, **caractérisé en ce qu'**il peut être déclenché électriquement.

5. Gazogène selon l'une des revendications précédentes, **caractérisé en ce qu'**il présente un dispositif de collecte pour du gaz de combustion généré, lequel présente au moins un moyen pour la fermeture.

6. Procédé pour contrôler un détecteur de gaz ou un détecteur de chaleur avec un gazogène selon l'une des revendications précédentes, dans lequel
a. au moins un gazogène disposé de manière décentralisée est activé à l'aide d'un dispositif de surveillance central,
b. le gazogène générant ce faisant un gaz, de préférence un gaz de combustion,
c. le détecteur de gaz détectant le gaz de combustion, et le détecteur étant activé ce faisant, et le détecteur de gaz transmettant un signal de données.
